# DEMANDE DE BREVET EUROPEEN

(11) **EP 1 025 782 A1**
(43) Date de publication de la demande: **09.08.2000**
(21) Numéro de dépôt: 00470002.7
(22) Date de dépôt: 02.02.2000
(51) Int. Cl.: A47G 9/00, A47C 16/00, A61F 5/56

(54) **Oreiller anatomique multifonctions**

(30) Priorité: 03.02.1999 FR 9901334
(71) Demandeur: Marchal, Jean-Luc, 88000 Epinal (FR)
(72) Inventeur: Marchal, Jean-Luc, 88000 Epinal (FR)
(74) Mandataire: Poupon, Michel

(57) **Abrégé**

Oreiller anatomique multifonctions permettant de soulager les douleurs et déformations vertébrales grâce au maintien d'une position idéale de la tête ainsi que d'empêcher les ronflements pendant le sommeil, qui se compose essentiellement d'une coquille de maintien monobloc semi-rigide composée de deux ailes (4) de protections latérales reliées entre elles par une section (2,3) incurvée formant zone d'appui pour le maintien de la nuque en décubitus dorsal, la coquille étant solidarisée à la tête de l'utilisateur par une sangle (7) de maintien réglable disposée sensiblement dans un plan parallèle à l'axe vertical de l'utilisateur.

## Description

La présente invention a pour objet un oreiller anatomique multifonctions permettant de soulager les douleurs et déformations vertébrales grâce au maintien d'une position idéale de la tête ainsi que d'empêcher les ronflements pendant le sommeil, du type comportant une pluralité d'éléments souples reliés uniquement entre eux, enserrant la tête au moyen de lanières ou de pattes d'attache et rendus solidaires de celle-ci au moyen d'attaches rapides ou de lanières.

Les oreillers anatomiques traditionnels nécessitent une parfaite adaptation entre la tête et l'oreiller. Lorsque l'utilisateur bouge pendant son sommeil, sa tête se trouve décalée par rapport à l'oreiller, le rendant ainsi inefficace.

De même on a proposé par le brevet US-A-4 679 263 un oreiller composé de trois coussins indépendants articulés entre eux par des sections planes formant charnières. Ce dispositif est de structure complexe, donc onéreux, et n'est pas assuré d'un maintien optimal sur la tête de l'utilisateur.

Le brevet US-A-4 550 458 est d'une conception différente : le coussin est destiné à venir se bloquer par sa partie inférieure sur les épaules de l'utilisateur empêchant ainsi tout mouvement de tête de la part dudit utilisateur. Il utilise en outre un moyen de fixation complexe et inadéquat.

Enfin, le brevet US-A-5 378 042 concerne un dispositif de support de tête, comportant une pluralité de coussins, dispositif essentiellement destiné au blocage de la tête de l'utilisateur par une sangle frontale.

L'invention a pour but de remédier aux inconvénients des dispositifs de l'art antérieur en proposant un oreiller anatomique mutlifonctions permettant le maintien en bonne position de la tête de l'utilisateur pendant le sommeil pour soulager les douleurs et éviter les déformations vertébrales.

Conformément à l'invention, ce résultat est obtenu avec un oreiller anatomique multifonctions permettant de soulager les douleurs et déformations vertébrales grâce au maintien d'une position idéale de la tête ainsi que d'empêcher les ronflements pendant le sommeil, du type comportant une pluralité d'éléments souples reliés uniquement entre eux, enserrant la tête au moyen de lanières ou de pattes d'attache et rendus solidaires de celle-ci au moyen d'attaches rapides ou de lanières, caractérisé en ce qu'il se compose essentiellement d'une coquille de maintien monobloc semi-rigide composée de deux ailes de protections latérales reliées entre elles par une section incurvée formant zone d'appui pour le maintien de la nuque en décubitus dorsal, la coquille étant solidarisée à la tête de l'utilisateur par une sangle de maintien réglable disposée sensiblement dans un plan parallèle à l'axe vertical de l'utilisateur.

Principalement, l'oreiller selon l'invention est constitué d'une structure incurvée en forme de gouttière de façon à envelopper la tête et d'une sangle de fixation assurant son maintien. La zone d'appui centrale, destinée à soutenir la nuque en décubitus dorsal, présente un rembourrage interne et un prolongement cervical permettant d'épouser la courbure nucale. Les plaques d'appui latérales destinées à soutenir la tête en décubitus latéral sont perforées de multiples trous et présentent une épaisseur adaptée à la largeur d'épaule de l'utilisateur. Elles sont traversées par une sangle de fixation et accessoirement par une sangle de blocage. La coquille sera avantageusement disposée dans une taie ou housse. La taie peut comporter une poche de réservation pouvant contenir des dispositifs anti-douleur.

Ainsi, grâce à sa forme générale en gouttière et à sa sangle de fixation, la mobilité acquise par l'oreiller lui assure une adaptation parfaite quels que soient les mouvements de la tête pendant le sommeil.

Selon des variantes de mise en oeuvre :
- la sangle de fixation peut être dédoublée dans sa partie supérieure et équipée d'une mentonnière anti-ronflement,
- la sangle de blocage peut être équipée d'un dispositif anti-ronflement complémentaire,
- les bords postérieurs des plaques d'appui latérales peuvent être taillés en biseau,
- la structure en gouttière peut être gonflable en totalité ou en partie.

On comprendra mieux l'invention à l'aide des figures annexées dans lesquelles :
- la figure 1 représente une perspective de l'oreiller vu de haut, d'arrière et de côté,
- la figure 2 représente une coupe de profil montrant le rembourrage interne de l'oreiller au contact de la nuque,
- la figure 3 représente une vue de profil montrant le positionnement de l'oreiller et de sa sangle de fixation selon une variante,
- la figure 4 représente une perspective de l'oreiller vu de haut, d'arrière et de côté, montrant le dispositif anti-ronflement et sa sangle de blocage.

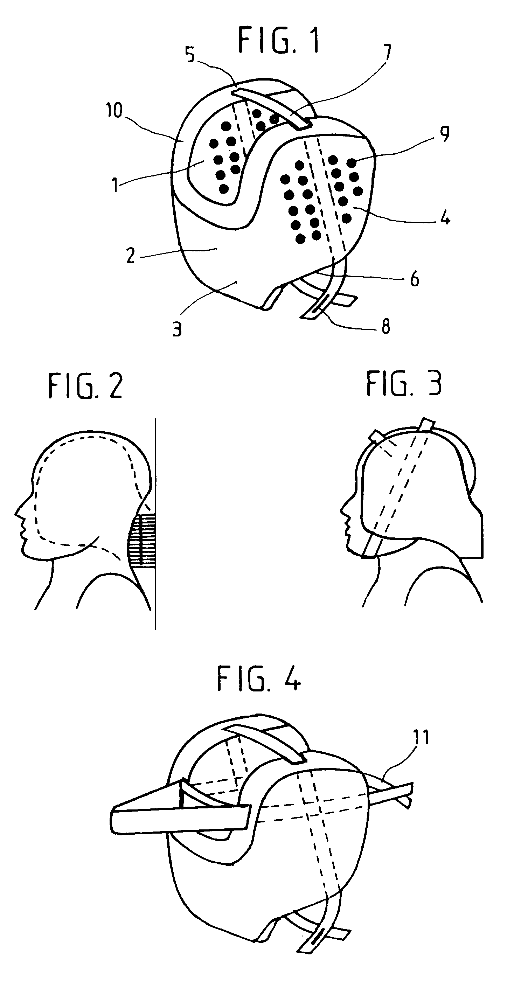

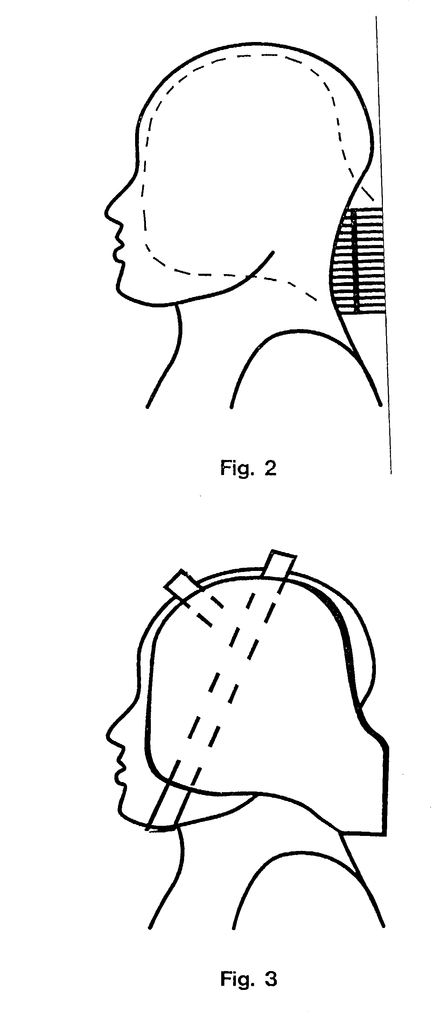

En référence à ces dessins, l'oreiller est constitué d'une structure de mousse synthétique incurvée en forme de gouttière particulièrement bien adaptée à la configuration anatomique de la région cervico-cranienne. La partie supérieure de la zone cintrée (1) est découpée de façon à recevoir l'arrière de la tête. La partie inférieure de la zone cintrée que l'on nommera « zone d'appui centrale » (2) comporte un rembourrage interne (figure 2) ainsi qu'un prolongement cervical (3) de façon à épouser les courbures de la nuque. Cette partie soutient la nuque en décubitus dorsal. Les deux parties latérales que l'on nommera « plaques d'appui latérales » (4) présentent une épaisseur adaptée à la largeur des épaules de façon à soutenir la tête en décubitus latéral. Elles sont fendues dans leur épaisseur sur quelques centimètres de large selon un axe rejoignant le milieu du bord supérieur (5) à la partie antérieure du bord inférieur (6). Dans cette fente coulisse une sangle dite « sangle de fixation » (7) qui bloque l'oreiller vers le haut, le bas et les côtés, lui assurant la mobilité indispensable à sa fonction anatomique lors des changements de position nocturne. L'extrémité inférieure de cette sangle garnie d'une bande « velcro » (8) permet non seulement la fixation de l'oreiller mais aussi le réglage de l'espace d'aération du visage en refermant plus ou moins les plaques d'appui latérales (4) qui ont tendance à s'écarter spontanément du fait de l'élasticité de la mousse. La partie centrale des plaques d'appui latérales (4) est perforée de nombreux trous (9) diminuant ainsi la densité de la mousse et améliorant l'aération pour le confort du visage.

La taie comporte dans sa partie interne en vis-à-vis de la zone d'appui centrale (2) une poche de réservation pouvant contenir un dispositif de traitement des douleurs par réflexo-thérapie (coussin d'air massant, petites billes de bois, aimants).

Selon une variante de mise en oeuvre, la sangle peut être simplement disposée entre la coquille et la housse.

Selon une variante illustrée par la figure 3, la sangle de fixation peut être dédoublée dans sa partie supérieure, de manière à former une sangle frontale destinée à améliorer le blocage de l'oreiller vers l'avant.

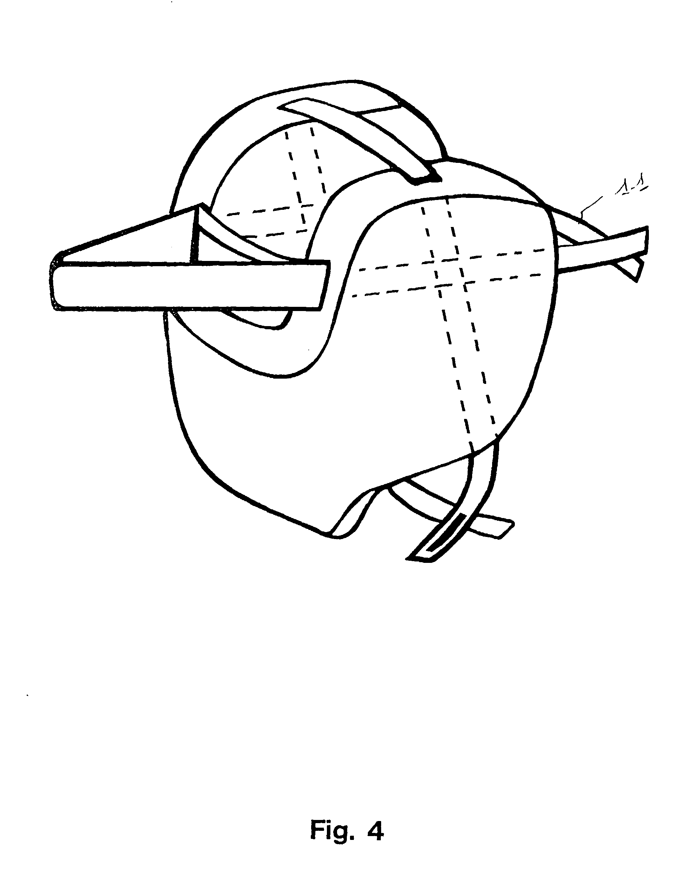

Selon une variante illustrée par la figure 4, une sangle (11) dite « sangle de blocage » coulisse dans une fente selon un axe antéropostérieur situé à l'union tiers supérieur deux tiers inférieur des plaques d'appui latérales. Cette sangle, garnie d'une bande « velcro » dans sa partie antérieure améliore le blocage de l'oreiller vers l'avant. Elle peut être équipée dans sa partie postérieure d'un dispositif anti ronflement empêchant de dormir sur le dos. Il consiste en une armature triangulaire dont la base incurvée maintenue par la sangle vient s'appuyer sur l'occiput, rendant instable l'équilibre de la tête en décubitus dorsal et obligeant l'utilisateur à dormir sur le côté. L'armature peut être rendue amovible par une attache rapide.

Selon des variantes non illustrées :
- la sangle de fixation (7) peut être équipée d'une mentonnière anti-ronflement empêchant l'ouverture de la bouche pendant le sommeil,
- les angles de chaque bord postérieur (10) des plaques d'appui latérales (4) peuvent être taillés en biseau sur leur côté interne et externe de façon à favoriser la rotation de la tête respectivement selon le mode d'utilisation décrit et selon un mode d'utilisation traditionnelle une fois l'oreiller déplié par ablation de la sangle de fixation (7),
- la structure de l'oreiller peut être gonflable en totalité pour une utilisation « voyage » ou en partie pour une utilisation permettant de dormir assis par la mise en place d'un coussin gonflable. Ce coussin replié le long du bord inférieur (6) de chaque plaque d'appui latérale (4) permet, une fois gonflé, de caler l'oreiller sur les épaules, ce qui a pour effet d'empêcher la tête de basculer.

L'oreiller anatomique selon l'invention est particulièrement destiné à un usage médical : il soulage les douleurs et déformations cervicales, empêche les ronflements et atténue les allergies aux acariens en évitant tout contact des narines avec la literie. Il peut en outre être adapté à un usage de loisir et de voyage dans une variante équipée d'une structure gonflable offrant notamment la possibilité de dormir assis. Enfin, ce type d'oreiller peut facilement être réalisé sur mesure grâce aux mensurations du périmètre cranien, de la largeur d'épaule et de la flèche cervicale. Ces données peuvent être combinées à une photo de profil de l'ensemble tête-nuque offrant le gabarit idéal pour la fabrication de l'oreiller.

## Revendications

1. Oreiller anatomique multifonctions permettant de soulager les douleurs et déformations vertébrales grâce au maintien d'une position idéale de la tête ainsi que d'empêcher les ronflements pendant le sommeil, du type comportant une pluralité d'éléments souples reliés uniquement entre eux, enserrant la tête au moyen de lanières ou de pattes d'attache et rendus solidaires de celle-ci au moyen d'attaches rapides ou de lanières, caractérisé en ce qu'il se compose essentiellement d'une coquille de maintien monobloc semi-rigide composée de deux ailes de protections latérales reliées entre elles par une section incurvée formant zone d'appui pour le maintien de la nuque en décubitus dorsal, la coquille étant solidarisée à la tête de l'utilisateur par une sangle de maintien réglable disposée sensiblement dans un plan parallèle à l'axe vertical de l'utilisateur.

2. Oreiller selon la revendication 1, caractérisé en ce que la « sangle de fixation » (7) comporte à son extrémité inférieure une bande « velcro »(8) permettant à la fois la fixation de l'oreiller, le réglage de son espace d'aération et l'adaptation d'une mentonnière anti-ronflement.

3. Oreiller selon l'une quelconque des revendications 1 et 2, caractérisé en ce qu'il comporte une zone d'appui centrale (2) destinée à soutenir la nuque en décubitus dorsal grâce à son rembourrage interne et à son prolongement cervical (3).

4. Oreiller anatomique selon l'une quelconque des revendications 1 à 3, caractérisé en ce qu'il comporte des plaques d'appui latérales (4) perforées de trous (9) diminuant la densité de la mousse et améliorant l'aération pour le confort du visage.

5. Oreiller anatomique selon la revendication 4, caractérisé en ce que les angles de chaque bord postérieur (10) des plaques d'appui latérales (4) sont taillés en biseau sur leur côté interne et externe de façon à favoriser la rotation de la tête.

6. Oreiller anatomique selon l'une quelconque des revendications 1 à 5, caractérisé en ce qu'il est enveloppé dans une taie en tissu.

7. Oreiller selon la revendication 6, caractérisé en ce que la taie en tissu comporte sur sa face interne une poche de réservation en regard de la zone d'appui centrale (2).

8. Oreiller anatomique selon la revendication 1, caractérisé en ce qu'il comporte une sangle supplémentaire de blocage (11).

9. Oreiller anatomique selon la revendication 8, caractérisé en ce que la sangle de blocage est équipée d'une armature triangulaire anti-ronflement dont la base incurvée vient s'appuyer sur l'occiput de l'utilisateur.

10. Oreiller anatomique selon l'une quelconque des revendications 1 à 9, caractérisé en ce que la structure en gouttière qui le constitue est gonflable en partie ou en totalité.
